(19) Europäisches Patentamt / European Patent Office / Office européen des brevets

(11) **EP 3 103 378 A1**

(12) **EUROPEAN PATENT APPLICATION**
published in accordance with Art. 153(4) EPC

(43) Date of publication:
**14.12.2016 Bulletin 2016/50**

(21) Application number: **15776543.9**

(22) Date of filing: **31.03.2015**

(51) Int Cl.:
*A61B 1/04* (2006.01)   *A61B 1/00* (2006.01)
*G02B 23/24* (2006.01)

(86) International application number:
**PCT/JP2015/060189**

(87) International publication number:
**WO 2015/156176 (15.10.2015 Gazette 2015/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **08.04.2014 JP 2014079766**

(71) Applicant: **Olympus Corporation
Tokyo 192-8507 (JP)**

(72) Inventor: **HONDA Kazuki
Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(54) **ENDOSCOPE SYSTEM**

(57)    An endoscope system includes an insertion section 4, an image pickup section 34 provided in the insertion section 4, a first optical system 12 configured to project a first subject image onto the image pickup section 34, a second optical system 13 configured to project a second subject image onto the image pickup section, an image generating section 60 configured to generate an image based on the first subject image and the second subject image, a storing section 64 configured to record information for setting a position of a boundary between the first subject image and the second subject image, and an image processing section 61 configured to perform predetermined image processing on the boundary between the first subject image and the second subject image based on the information for setting the position of the boundary.

## FIG. 5

# FIG. 6

**Description**

Technical Field

The present invention relates to an endoscope system and, more particularly, to an endoscope system capable of independently and simultaneously observing a front visual field and a side visual field.

Background Art

An endoscope system including an endoscope that picks up an image of a subject on an inside of a lumen such as a body cavity and an image processing apparatus that generates an observation image of the subject, the image of which is picked up by the endoscope, is widely used in a medical field, an industrial field, and the like.

For example, Japanese Patent No. 5030675 discloses an endoscope that simultaneously forms a front visual field image and a side visual field image on one image pickup device and obtains a wide-angle visual field image.

Japanese Patent No. 4856286 and Japanese Patent No. 4884567 disclose a wide-angle endoscope that distinguishes a front visual field region and a side visual field region and performs image processing in the respective regions.

Japanese Patent No. 4856286 discloses a technique for performing gain adjustment for the respective visual fields of a front visual field image (front visual field image) and a side visual field image (side visual field image) for the purpose of visibility improvement. Japanese Patent No. 4884567 discloses a technique for performing enlargement/reduction processing of the respective visual fields for each scene for the same purpose of visibility improvement.

On the other hand, Japanese Patent Application Laid-Open Publication No. 2013-066646 discloses image processing for clarifying a boundary portion between a front visual field image and a side visual field image for the purpose of visibility improvement.

As disclosed in Japanese Patent No. 5030675, Japanese Patent No. 4856286, Japanese Patent No. 4884567, and Japanese Patent Application Laid-Open Publication No. 2013-066646, in the endoscope that generates a wide-angle image from visual fields in a plurality of directions, it is likely that, when an image pickup unit is assembled, axis deviation occurs between an optical axis of an objective optical system and a center axis of an image pickup device because of influence of machining accuracy of frames of the objective optical system and the image pickup device, irregularity during the assembly, or the like.

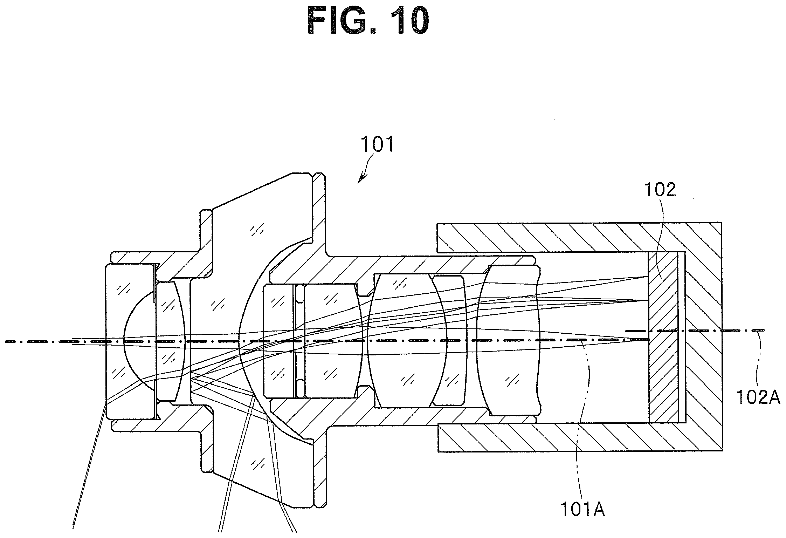

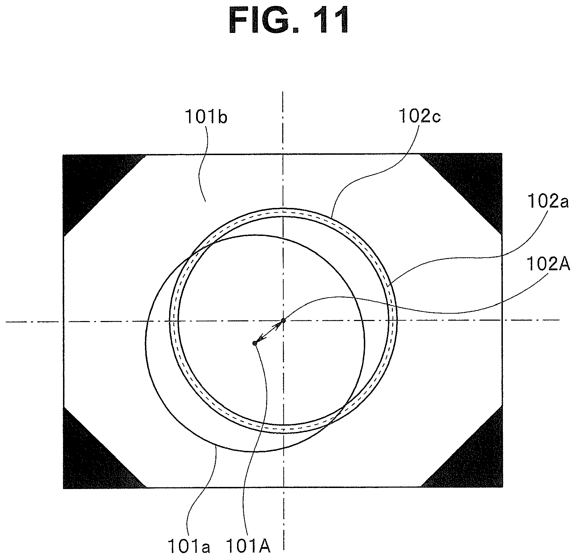

Fig. 10 is a main part sectional view showing a configuration example of an image pickup unit disposed in an insertion-section distal end portion in a conventional wide-angle endoscope. Fig. 11 is a diagram for explaining deviation of a visual field region in the conventional wide-angle endoscope.

More specifically, in an endoscope that generates a wide-angle image from visual fields in a plurality of directions, for example, as shown in Fig. 10, it is likely that axis deviation occurs between an optical axis 101A of an objective optical system 101 and a center axis 102A of an image pickup device 102 because of influence of assembly machining accuracy, irregularity, or the like of an image pickup unit.

As shown in Fig. 11, when the axis deviation occurs between the optical axis 101A of the objective optical system and the center axis 102A of the image pickup device, since an image processing range 102c set in advance in a processor, which performs image processing, assuming that a boundary 102a of a visual field region (a boundary between a front visual field region and a side visual field region 101b) is located in the image processing range 102c and a boundary 101a of an actual visual field region are different, it is likely that a range in which the image processing is performed deviates and the image processing cannot be accurately performed.

That is, in the image pickup unit shown in Fig. 10, in the objective optical system, a lens is fit in a cylindrical frame to perform positioning of a lens center. Even if accuracy of the frame is strictly set, if an error such as irregularity occurs in addition to deviation of machining accuracy during assembly, a position where an image is projected deviates with respect to a targeted position of the image pickup device.

This causes a deficiency that, for example, image processing for making a boundary between a front visual field region and a side visual field region less noticeable cannot be performed in an accurate range.

The deficiency is conspicuous in an endoscope that simultaneously displays a front visual field region and a side visual field region. Even if machining accuracy is refined and precise assembly is performed (even if assembly precision is in a level of several micrometers), deviation is sometimes extremely noticeable when the front visual field region and the side visual field region are displayed on a monitor screen.

The present invention has been devised in view of the circumstances described above and an object of the present invention is to provide an endoscope system capable of independently and simultaneously observing a front visual field and a side visual field, the endoscope system being capable of precisely performing image processing even when axis deviation occurs between an optical axis of an objective optical system and a center axis of an image pickup device.

Disclosure of Invention

Means for Solving the Problem

An endoscope system according to an aspect of the present invention includes: an insertion section inserted into an inside of a subject; an image pickup section provided in the insertion section; a first optical system provided in the insertion section and configured to project a first subject image concerning a first region of the subject onto the image pickup section; a second optical system provided in the insertion section and configured to project a second subject image concerning a second region of the subject, at least a part of which is different from the first region, onto the image pickup section; an image generating section configured to generate an image based on the first subject image and the second subject image projected onto the image pickup section; a storing section configured to record information for setting a position of a boundary between the first subject image and the second subject image in the image; and an image processing section configured to perform predetermined image processing on the boundary between the first subject image and the second subject image based on the information for setting the position of the boundary.

Brief Description of the Drawings

Fig. 1 is a diagram showing a configuration of an endoscope system in a first embodiment of the present invention;

Fig. 2 is a perspective view showing a configuration of an insertion-section distal end portion in the endoscope system in the first embodiment;

Fig. 3 is a main part sectional view showing the configuration of the insertion-section distal end portion in the endoscope system in the first embodiment;

Fig. 4 is a diagram showing an overview of an observation image displayed on a monitor screen in the endoscope system in the first embodiment;

Fig. 5 is a block diagram showing a main part of an electric configuration of an endoscope and a processor in the endoscope system in the first embodiment;

Fig. 6 is a diagram showing an example of the observation image displayed on the monitor screen in the endoscope system in the first embodiment;

Fig. 7 is a block diagram showing an electric main part configuration of an endoscope and a processor in an endoscope system in a second embodiment of the present invention;

Fig. 8 is a diagram showing an overview of an observation image displayed on a monitor screen in the endoscope system in the second embodiment;

Fig. 9 is a diagram showing an example of the observation image displayed on the monitor screen in the endoscope system in the second embodiment;

Fig. 10 is a main part sectional view showing a configuration example of an image pickup unit disposed at an insertion-section distal end portion in a conventional wide-angle endoscope; and

Fig. 11 is a diagram for explaining deviation of a visual field region in the conventional wide-angle endoscope.

Best Mode for Carrying Out the Invention

Embodiments of the present invention are explained below with reference to the drawings.

(First Embodiment)

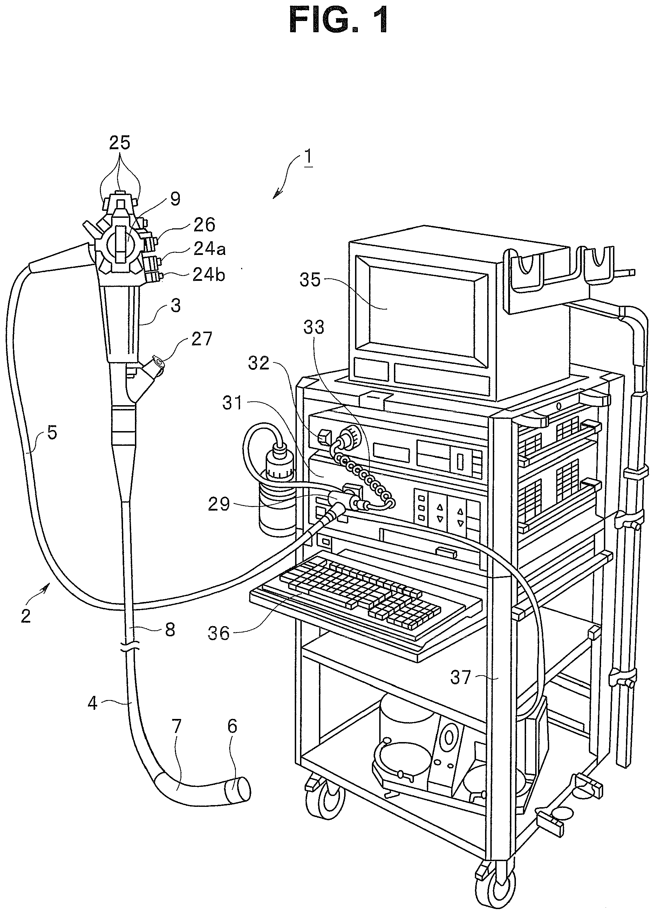

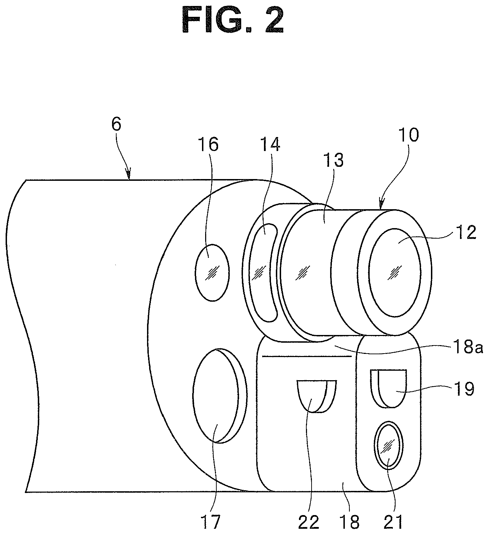

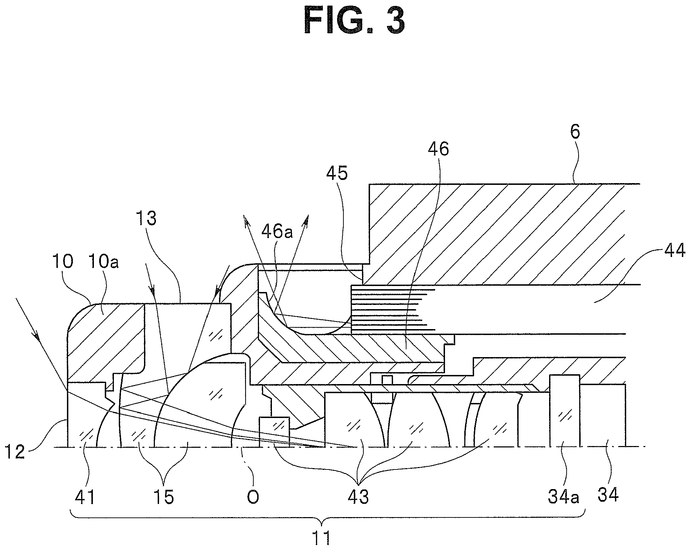

A configuration of an endoscope system in a first embodiment is explained with reference to Fig. 1 to Fig. 3. Fig. 1 is a diagram showing the configuration of the endoscope system in the first embodiment of the present invention. Fig. 2 is a perspective view showing a configuration of an insertion-section distal end portion in the endoscope system in the first embodiment. Fig. 3 is a main part sectional view showing the configuration of the insertion-section distal end portion in the endoscope system in the first embodiment.

As shown in Fig. 1, an endoscope system 1 includes an endoscope 2 that picks up an image of an observation target object and outputs an image pickup signal, a light source apparatus 31 that supplies illumination light for illuminating the observation target object, a video processor 32 that generates and outputs a video signal corresponding to the image pickup signal, and a monitor 35 that displays an observation image corresponding to the video signal.

The endoscope 2 includes an operation section 3 that a surgeon grasps to perform operation, an elongated insertion section 4 formed on a distal end side of the operation section 3 and inserted into a body cavity or the like, and a universal cord 5, one end portion of which is provided to extend from a side portion of the operation section 3.

The endoscope 2 in the present embodiment is a wide-angle endoscope capable of observing a visual field equal to or larger than 180 degrees. The endoscope 2 realizes prevention of overlooking of a lesion in a place hardly seen by only observation in a front direction such as a back of a fold or a boundary of an organ in a body cavity, in particular, in a large intestine. In inserting the insertion section 4 of the endoscope 2 into the large intestine, as in a normal large intestine endoscope, an action such as provisional fixing is generated in the insertion section 4 by performing twisting, a reciprocating motion, and hook of an intestinal wall.

The insertion section 4 includes a rigid distal end portion 6 provided at a most distal end side, a bendable bending section 7 provided at a rear end of the distal end portion 6, and a long flexible tube section 8 having flexibility provided at a rear end of the bending section 7. The bending section 7 performs a bending action corresponding to operation of a bending operation lever 9 provided in the operation section 3.

As shown in Fig. 2, a cylindrical section 10 is formed at the distal end portion 6 of the insertion section 4 as a cylindrical member projecting in a cylindrical shape from a position decentered to, for example, an upper side from a center of a distal end face of the distal end portion 6. The cylindrical section 10 is provided at a distal end of the insertion section 4, includes a distal end face smaller in diameter than (an outer diameter of) the insertion section 4 and facing an inserting direction and an outer circumference side surface facing a circumferential direction of the insertion section 4, and forms a distal end portion in which a front observation window 12, a side observation window 13, and the like explained below are provided.

A front observation window 12 functioning as a front observation section (a first optical system) and a side observation window 13 functioning as a side observation section (a second optical system) are formed on a distal end side of the cylindrical section 10 using an objective optical system 11 (see Fig. 3) functioning as both of a front objective optical system and a side objective optical system for performing optical observation. At least one side illumination window 14 is formed near a proximal end of the cylindrical section 10 as a side illumination section.

The front observation window 12 acquires a first subject image from a direct view direction (a first direction) including the front substantially parallel to a longitudinal direction of the insertion section 4, that is, a first region of a subject and projects the first subject image onto an image pickup device 34 (an image pickup section; see Fig. 3) explained below.

The side observation window 13 is formed in a ring shape to set an entire circumference thereof as an observation visual field along a circumferential direction of a side surface of a cylindrical shape (a circumferential direction of the insertion section 4) for observing a direction of the side surface.

That is, the side observation window 13 acquires a second subject image from a direction at least partially different from the longitudinal direction (the first region) of the insertion section 4 and in a side view direction (a second direction) including a side direction of the insertion section 4, that is, a second region of the subject.

The side observation window 13 includes a mirror lens 15 functioning as a reflection optical system for capturing, in a side observation visual field (simply referred to as visual field), light (a second subject image) from the subject made incident from any direction opposed to the ring shape and acquiring the light as a side visual field image. The side observation window 13 projects the acquired second subject image onto the image pickup device 34 (the image pickup section; see Fig. 3) explained below via the mirror lens 15.

A front illumination window 16 that is adjacent to the cylindrical section 10 and emits illumination light to an observation target side of a front visual field of the front observation window 12 and a channel-distal-end opening section 17 functioning as an opening for projecting a treatment instrument inserted through a channel are provided on the distal end face of the distal end portion 6. In the present embodiment, a supporting section 18 is provided adjacent to a lower part side of the cylindrical section 10 to project from the distal end face of the distal end portion 6.

The supporting section 18 has a function of optically blocking a projecting member, which is not an original observation target, projected from the distal end face not to appear in the side visual field to be acquired as a side visual field image. The supporting section 18 supports the cylindrical section 10.

The supporting section 18 supports a nozzle section for side observation window 22, a distal end of which projects to a side surface of the supporting section 18, opening toward the side observation window 13 to clean the side observation window 13. The supporting section 18 blocks the nozzle section for side observation window 22 not to appear in a side visual field image.

Referring back to Fig. 1, in the operation section 3, gas/liquid feeding operation buttons 24a and 24b are provided to make it possible to selectively eject gas and liquid for cleaning respectively from a nozzle section for front observation window 19 and the nozzle section for side observation window 22. Air feeding and liquid feeding can be switched by operation of the gas/liquid feeding operation buttons 24a and 24b.

In the operation section 3, a suction operation button 26 for sucking and collecting mucus and the like in the body cavity from the channel-distal-end opening section 17 is disposed. Note that the channel is formed by a not-shown tube or the like disposed in the insertion section 4. The channel communicates with a treatment-instrument insertion port 27 provided near a front end of the operation section 3.

When a surgeon intends to perform treatment by a treatment instrument, the surgeon can perform treatment for therapeutic curing by the treatment instrument by inserting the treatment instrument from the treatment-instrument insertion port 27 and projecting a distal end side of the treatment instrument from the channel-distal-end opening section 17.

A connector 29 is provided at an end of the universal cord 5. The connector 29 is connected to the light source apparatus 31 of the endoscope. A cap (not shown in the figure) functioning as a connection end portion of a fluid conduit projecting from a distal end of the connector 29 and a light guide cap (not shown in the figure) functioning as a supply end portion of illumination light are detachably connected to the light source apparatus 31. One end of the connection cable 33 is connected to an electric contact section provided on a side surface of the connector 29.

A connector at the other end of the connection cable 33 is electrically connected to the video processor 32 functioning as a signal processing apparatus that performs signal processing for the image pickup device 34 (the image pickup section; see Fig. 3) mounted on the endoscope 2.

Note that, in the connector 29, an IC chip 64, which is a storing section having stored therein peculiar predetermined ID information in the endoscope 2, is disposed (explained in detail below).

The video processor 32 supplies a driving signal for driving the image pickup device 34 (the image pickup section; see Fig. 3) mounted at the distal end portion 6 of the endoscope 2, performs signal processing on an image pickup signal (an image signal) outputted from the image pickup device 34 according to the supply of the driving signal, and generates a video signal.

The video signal generated by the video processor 32 is outputted to the monitor 35 functioning as a display apparatus. An image picked up by the image pickup device 34 is displayed on a display surface of the monitor 35 as an endoscopic image. Peripheral apparatuses such as the light source apparatus 31, the video processor 32, and the monitor 35 are disposed on a counter 37 together with a keyboard 36 for performing, for example, an input of patient information.

Illumination light generated by the light source apparatus 31 is guided (transmitted) to, through a light guide inserted through the operation section 3 and the insertion section 4 from the universal cord 5, a distal end face side of the light guide. The distal end face of the light guide inserted through the insertion section 4 is disposed in the side illumination window 14 of the cylindrical section 10 projecting from the distal end portion 6, the front illumination window 16, and a front illumination window 21 (provided in the supporting section 18). The distal end face emits the guided light.

Note that a distal end side of the light guide is divided, for example, in the insertion section 4. One of the divided distal ends functions as a light guide 44 in the side illumination window 14 and the other of the divided distal ends functions as a not-shown light guide in the front illumination windows 16 and 21.

The illumination light is emitted from the side illumination window 14 and the front illumination windows 16 and 21 to be respectively expanded to a side surface direction, which is a side visual field side, and a distal end side in an inserting direction (referred to as longitudinal direction as well) of the insertion section 6, which is a front visual field side, to illuminate an observation target side in the body cavity.

Note that an illumination-light emitting portion disposed in the front illumination windows 16 and 21 and the side illumination window 14 may be a light emitting element such as a light emitting diode (LED) instead of the light guide.

Fig. 3 is a main part sectional view showing a configuration of the insertion-section distal end portion in the endoscope system in the first embodiment. Fig. 3 shows a configuration of a peripheral section of the objective optical system 11, which functions as both of the front objective optical system and the side objective optical system, and the side illumination window 14.

A front lens 41, the mirror lens 15, and a rear lens group 43 respectively having rotation symmetrical shapes are disposed on an optical axis, which coincides with an image pickup center O along a center axis of the cylindrical section 10 projecting from the distal end portion 6, to form the objective optical system 11 that forms an image on the image pickup device 34. Note that a cover glass 34a is provided on a front surface of the image pickup device 34. The front lens 41, the mirror lens 15, and the rear lens group 43 are fixed to a lens frame in the cylindrical section 10.

The front lens 41 configuring the objective optical system 11 and provided in the circular front observation window 12 forms a wide-angle front visual field in which an observation visual field is a front end side of the front lens 41 along an inserting direction of the insertion section 4.

As shown in Fig. 3, the mirror lens 15 functioning as a reflection optical system disposed right behind the front lens 41 is configured by a lens obtained by bonding two lenses that reflect light, which is made incident from the side surface direction, twice on a bonded surface and a front surface and guide the light to the rear lens group 43 side.

Note that a lens portion opposed to the front lens 41 in the mirror lens 15 also has a function of diffracting the light from the front lens 41 and guiding the light to the rear lens group 43 side.

The side observation window 13 forms, with the mirror lens 15 provided in the side observation window 13, a substantially annular observation visual field that covers an entire circumference in an insertion section circumferential direction while having a predetermined visual field angle substantially centering on an optical axis in a side direction with respect to an insertion section major axis direction.

Note that, in Fig. 3, schematic routes of a ray made incident on the front lens 41, which forms the front observation window 12 (the first optical system), from a subject side in a visual field of the front lens 41 and a ray made incident on the mirror lens 15 (the second optical system), which forms the side observation window 13, from a subject side in a visual field of the mirror lens 15 are shown.

The ray on the subject side made incident from the front lens 41 and the ray from the subject side made incident from the mirror lens 15 are projected onto the image pickup device 34.

An image of the subject in a front visual field provided to face an inserting direction by the front lens 41 of the front observation window 12 on a center side thereof, that is, a first subject image is formed in a circular shape on an image pickup surface of the image pickup device 34. The first subject image is acquired as a front visual field image. On the image pickup surface, an image of the subject in a side visual field, that is, a second subject image is formed in an annular shape such as a ring shape on an outer circumference side of the front visual field image by the mirror lens 15 facing the side observation window 13. The second subject image is acquired as a side visual field image to be adjacent to the front visual field image.

That is, the image pickup surface of the image pickup device 34 is disposed to photoelectrically convert, on the same surface, the first subject image projected from the front lens 41 and the second subject image projected from the second optical system. The image pickup device 34 is electrically connected to an image generating section 60 explained below, which generates an endoscopic image, provided in the video processor 32.

However, in the present embodiment, a blocking section 18a that mechanically blocks light from the subject side made incident on the ring-shaped side visual field is formed by the supporting section 18. In the present embodiment, the side illumination light emitted from the side illumination window 14 side to the side surface direction is not emitted to the supporting section 18 side.

Note that, in the present embodiment, a twice-reflecting optical system that reflects return light twice on the mirror lens 15 is used. However, it is also possible to reflect the return light once with a once-reflecting optical system to form an image of the subject in the side visual field, process the image with the video processor 32, and align a side-view visual field image and a direct-view visual field image.

The side illumination windows 14 are provided in a plurality of places on an outer circumferential surface near a proximal end adjacent to the side observation window 13 in the cylindrical section 10. In the present embodiment, the side illumination windows 14 are provided in two places on both left and right sides in the circumferential direction. The side illumination windows 14 emit side illumination light to an entire region in the circumferential direction excluding a lower side where the supporting section 18 is provided.

A distal end side of the light guide 44 functioning as a light emitting member disposed along the longitudinal direction of the distal end portion 6 as shown in Fig. 3 is extended to near a proximal end of a cylindrical member 10a configuring the cylindrical section 10 projecting from the distal end face of the distal end portion 6.

Near the proximal end of the cylindrical section 10 (on an outer circumferential side of the rear lens group 43), a distal end face of the light guide 44 is disposed near a side surface of the cylindrical section 10. The distal end face of the light guide 44 functions as an emission end face that emits guided light. The distal end face of the light guide 44 emits the light in a distal end direction. In the present embodiment, the emission end face is circular. However, the emission end face is not limited to the circular shape and may have a deformed shape including an elliptical shape and a polygonal shape.

In a position that the emission end face faces, a recessed section 45a forming a light guide groove 45 functioning as a groove section that extends long in a belt shape along a side surface outer circumference of a cylindrical shape of the cylindrical section 10 centering on the position and guides light is provided. In the recessed section 45a, a reflection member 46 functioning as an illumination reflecting section formed to face the emission end face is disposed in the recessed section 45a. The light guide groove 45 provided with a reflecting section 46a that reflects light is formed on an inner surface of the reflection member 46.

The reflecting section 46a on an inner surface of the light guide groove 45 (formed by the reflection member 46) is a concave surface having a substantially semispherical shape on a longitudinal section shown in Fig. 3. The semispherical concave surface of the reflecting section 46a is formed longer than the emission end face of the light guide 44 along a circumferential direction of the cylindrical section 10.

The reflecting section 46a reflects light emitted from the emission end face toward the distal end side of the distal end portion 6 to change a traveling direction of the light to a side surface direction, guides the light to a side surface direction in a wide range along the circumferential direction to emit the light from the side illumination window 14, and illuminates an observation visual field side (an observation target side) of the side observation window 13. Therefore, the light emitted from the light guide groove 45 in the side surface direction is side illumination light.

Note that the reflecting section 46a can be formed by providing a metal thin film having high reflectance of aluminum, chrome, nickel chrome, silver, gold, or the like on the inner surface of the reflection member 46.

In this way, in the present embodiment, the reflection member 46 is disposed in the recessed section 45a such that the light guide groove 45 provided with the reflecting section 46a along the side surface outer circumference of the cylindrical section 10 is formed long. The emission end face of the light guide 44 functioning as the light emitting member is disposed to be located near a center position in the circumferential direction in the reflection member 46 (or the light guide groove 45).

The light emitted from the emission end face of the light guide 44 is reflected by the reflecting section 46a disposed to form a reflection surface around the emission end face. The illumination light is emitted to a wide range from the side illumination window 14, in which the light guide groove 45 is provided, to a side.

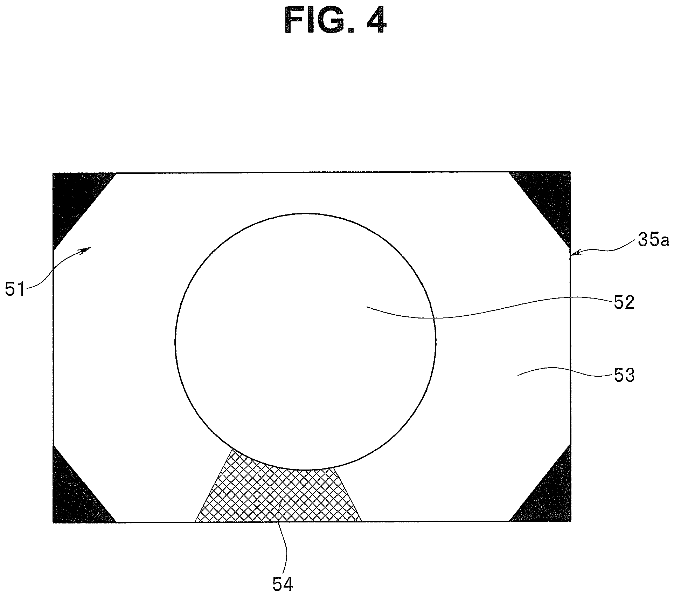

A display example in which a subject image picked up by the image pickup device 34 is displayed on a display surface 35a of the monitor 35 as an endoscopic image using the endoscope 2 is shown in Fig. 4.

A display region 51 in Fig. 4 corresponds to a display region of the image pickup surface of the image pickup device 34. A circular region in a center in the display region 51 is a display region 52 of the front visual field image by the front observation window 12. An annular region on an outer side of the display region 52 is a display region 53 of the side visual field image by the side observation window 13.

A region, a part on a lower side of which in the side visual field is blocked by the blocking section 18a formed in the supporting section 18, is a black region 54 in the side visual field image. That is, the black region 54 is a non-image output region in which an image is not displayed.

A main part of an electric configuration in the endoscope system in the first embodiment is explained.

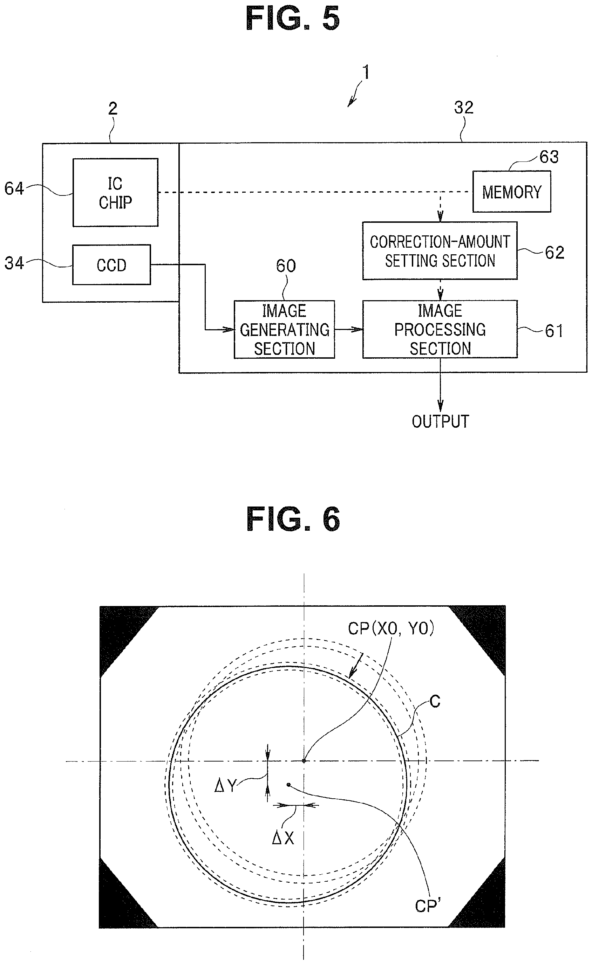

Fig. 5 is a block diagram showing a main part of an electric configuration of the endoscope and the processor in the endoscope system in the first embodiment.

In the endoscope system 1 in the first embodiment, the IC chip 64, which is the storing section, is disposed in, for example, the connector 29 in the endoscope 2. The IC chip 64, which is the storing section, is configured to store peculiar predetermined ID information in the endoscope 2, for example, in the present embodiment, position information of a boundary portion in the endoscope 2.

More specifically, the position information of the boundary portion is "a shift amount ($\Delta X$, $\Delta Y$) between a predetermined reference point and a front visual field center" set for each endoscope in advance before operation of the endoscope 2 on the basis of a state detected as a result of once displaying an endoscopic image, for example, during inspection before shipment after assembly of the endoscope 2 is performed.

On the other hand, the video processor 32 includes an image generating section 60 that receives an input of an image pickup signal from the image pickup device (the CCD) 34 and generates an endoscopic image, an image processing section 61 that applies predetermined image processing to the generated endoscopic image, a memory 63 that stores a coordinate (X0, Y0) of a reference point CP of the image processing in the video processor 32, and a correction-amount setting section 62 having a function of a position detecting section for setting a predetermined correction reference point on the basis of the position information of the boundary portion in the endoscope 2 acquired from the IC chip 64, which is the storing section, in the connector 29 and the reference point information stored in the memory 63.

Note that, in Fig. 5, arrows of a solid line connecting respective blocks indicate a flow of an image signal. Arrows of a broken line indicate a flow of predetermined parameters.

Processing by the image processing section 61 and the correction-amount setting section 62 in the first embodiment is explained with reference to Fig. 5 and Fig. 6.

Fig. 6 is a diagram showing an example of an observation image displayed on the monitor screen in the endoscope system 1 in the first embodiment.

Note that the black region 54 (see Fig. 4) is the non-image output region where an image is not displayed as explained above. In the following explanation, the black region 54 is omitted on the drawings such as Fig. 6.

First, after performing assembly of the endoscope 2, an endoscopic image is generated based on an image pickup signal from the image pickup device 34 using the video processor 32 connected to the endoscope 2 during inspection before shipment and the endoscope system 1 is caused to once output the endoscopic image to display a front visual field image and a side visual field image.

Subsequently, the correction-amount setting section 62 reads "the coordinate (X0, Y0) of the reference point CP of the image processing" as the first position, which is a portion set in the image pickup region of the image pickup device 34 of the endoscope 2, set in the video processor 32 stored in the memory 63.

In the video processor 32, for example, in the memory 63, for example, information to the effect that image processing is performed on an annular region C, which is a frame-like designated range centering on the coordinate (X0, Y0) of the reference point CP of the image processing, serving as an image processing region in which the image processing is performed is stored in advance. The annular region C has width considered appropriate for performing the image processing of a boundary portion between a front visual field region and a side visual field region explained below.

Thereafter, the correction-amount setting section 62 functions as a position detecting section and detects, from the endoscopic image, "a relative shift amount ($\Delta X$, $\Delta Y$) between a predetermined reference point and a front visual field center" in the second endoscope 2, which is a position where the first subject image is formed within the image pickup region of the image pickup section serving as a second position.

On the basis of this result, the correction-amount setting section 62 having a function of the position detecting section detects information concerning a relative positional relation, that is, information concerning the relative shift amount ($\Delta X$, $\Delta Y$) between the predetermined reference point and the front visual field center for setting a position of the boundary portion between the front visual field region and the side visual field region or information concerning a position of the boundary portion between the front visual field region and the side visual field region calculated on the basis of ($\Delta X$, $\Delta Y$), generates a positional relation detection signal concerning a detected result, transmits the positional relation detection signal to the IC chip 64, which is the storing section, and causes the IC chip 64 to store the positional relation detection signal.

Thereafter, when the endoscope 2 is used on a separate occasion, first, the correction-amount setting section 62 reads the information concerning the relative shift amount ($\Delta X$, $\Delta Y$) between the predetermined reference point and the front visual field center or the information concerning the position of the boundary portion between the front visual field region and the side visual field region based on ($\Delta$X, $\Delta$Y) stored in the IC chip 64, which is the storing section.

Thereafter, the correction-amount setting section 62 sets a correction reference point CP' (X0+$\Delta$X, Y0+$\Delta$Y) on the basis of the coordinate (X0, Y0) of the reference point CP and the read "relative shift amount ($\Delta$X, $\Delta$Y) between the predetermined reference point and the front visual field center".

The image processing section 61 performs, on the basis of the correction reference point CP' (X0+$\Delta$X, Y0+$\Delta$Y) set by the correction-amount setting section 62, processing for, for example, moving a predetermined annular region, where image processing is performed, centering on the coordinate (X0, Y0) of the reference point CP of the image processing to an annular region centering on the coordinate (X0+$\Delta$X, Y0+$\Delta$Y) of CP'.

Thereafter, the image processing section 61 applies clarified image processing to the boundary portion between the front visual field region and the side visual field region using a publicly-known method in Japanese Patent Application Laid-Open Publication No. 2013-066646 laid open by the applicant. The image processing section 61 performs predetermined image processing such as processing for extending respective parts of a first object-subject image and the second subject image and filling and reducing a frame-like edge formed in the boundary between the first subject image and the second subject image in the respective regions (the front visual field region and the side visual field region) on the basis of the correction reference point CP' (X0+$\Delta$X, Y0+$\Delta$Y) using, for example, the method in Japanese Patent Application Laid-Open Publication No. 2013-066646.

As explained above, with the endoscope system in the first embodiment, it is possible to provide an endoscope system capable of independently and simultaneously observing a front visual field and a side visual field, the endoscope system being capable of precisely performing image processing even when axis deviation occurs between an optical axis of an objective optical system and a center axis of an image pickup device when an image pickup unit is assembled because of influence of machining accuracy of frames of the objective optical system and the image pickup device, irregularity during the assembly, or the like.

Note that a series of operations in which the endoscope system 1 is caused to once output the endoscopic image and the correction-amount setting section 62 reads (X0, Y0) stored in the memory 63, detects ($\Delta$X, $\Delta$Y) from the endoscopic image, and causes the IC chip 64, which is the storing section, to store ($\Delta$X, $\Delta$Y) is not limited to an example in which the operation is performed only during inspection before shipment of the endoscope 2.

As another method, a configuration may be adopted in which, every time the user turns on a power supply of the endoscope system 1 and causes the endoscope system 1 to output an endoscopic image, the correction-amount setting section 62 sets ($\Delta$X, $\Delta$Y) from an endoscopic image outputted first and causes the IC chip 64 to store ($\Delta$X, $\Delta$Y).

(Second Embodiment)

A second embodiment of the present invention is explained.

A basic configuration of an endoscope system in the second embodiment of the present invention is the same as the basic configuration in the first embodiment. However, correction processing and image processing in the video processor 32 are different compared with the first embodiment.

Therefore, only differences from the first embodiment are explained. Explanation concerning similarities to the first embodiment is omitted.

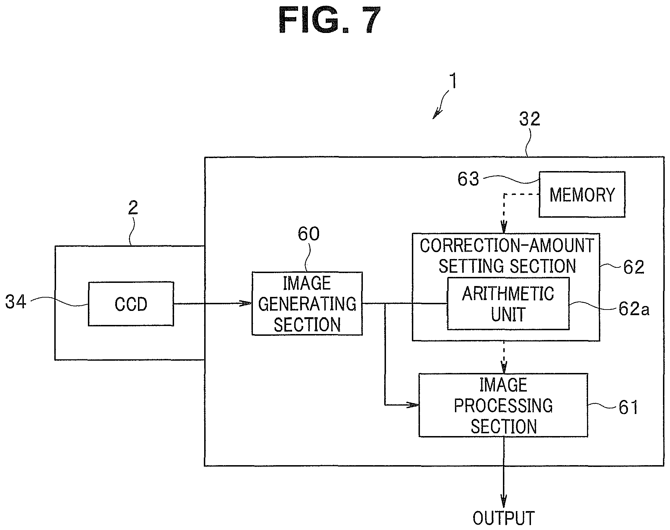

Fig. 7 is a block diagram showing a main part of an electric configuration of an endoscope and a processor in the endoscope system in the second embodiment.

The video processor 32 in the second embodiment includes the image generating section 60 that receives an input of an image pickup signal from the image pickup device (the CCD) 34, receives an input of an image pickup signal from the image pickup device (the CCD) 34, and generates an endoscopic image, the image processing section 61 that applies predetermined image processing to the generated endoscopic image, the memory 63 that stores a coordinate (X0, Y0) of a reference point CP of the image processing in the video processor 32, an arithmetic unit 62a that detects a so-called black solid pixel on the endoscopic image and calculates "a shift amount ($\Delta$X, $\Delta$Y) between a predetermined reference point and a front visual field center" on the basis of position information of the black solid pixel, and the correction-amount setting section 62 having a function of a position detecting section for setting a predetermined correction reference point on the basis of the shift amount ($\Delta$X, $\Delta$Y) and the coordinate (X0, Y0) of the reference point CP stored in the memory 63.

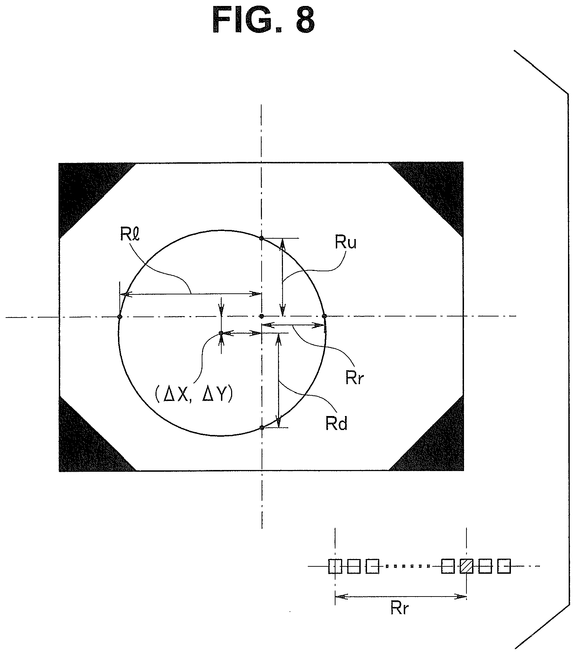

Processing by the image processing section 61 and the correction-amount setting section 62 (including the arithmetic unit 62a) in the second embodiment is explained with reference to Fig. 7 and Fig. 8.

Fig. 8 is a diagram showing an example of an observation image displayed on a monitor screen in the endoscope system in the second embodiment.

Note that black solid optically or electrically occurs in a boundary (reference sign 101a shown in Fig. 11) of a visual field region.

First, in use of the endoscope system 1, the endoscope system 1 is caused to once output an endoscopic image using the video processor 32 connected to the endoscope 2 to display a front visual field image and a side visual field image.

Subsequently, the correction-amount setting section 62 reads "the coordinate (X0, Y0) of the reference point CP of the image processing" as the first position, which is a portion set in the image pickup region of the image pickup device 34 of the endoscope 2, set in the video processor 32 stored in the memory 63.

In the video processor 32, for example, in the memory 63, for example, information to the effect that image processing is performed on an annular region, which is a frame-like designated range centering on the coordinate (X0, Y0) of the reference point CP of the image processing, serving as an image processing region in which the image processing is performed is stored in advance. The annular region has width considered appropriate for performing the image processing of a boundary portion between a front visual field region and a side visual field region explained below.

Thereafter, the correction-amount setting section 62 calculates "a shift amount ($\Delta$X, $\Delta$Y) between a predetermined reference point and a front visual field center" in the arithmetic unit 62a in the correction-amount setting section 62 from an endoscopic image outputted first when the user turns on the power supply of the endoscope system 1 and causes the endoscope system 1 to output an endoscopic image.

That is, more specifically, the arithmetic unit 62a calculates the numbers of pixels Ru, Rd, Rr, and Rl (see Fig. 8) from the reference point CP to black solid pixels on the X axis and the Y axis and calculates, for example, according to the following calculation formula, a shift amount ($\Delta$X, $\Delta$Y) between the reference point and the visual field center from values of the numbers of pixels.

$$\Delta X = |(Rr\text{-}Rl)/2|$$

$$\Delta Y = |(Ru\text{-}Rd)/2|$$

Thereafter, as in the first embodiment, the correction-amount setting section 62 sets the correction reference point CP' (X0+$\Delta$X, Y0+$\Delta$Y) on the basis of the coordinate (X0, Y0) of the reference point CP and the "shift amount ($\Delta$X, $\Delta$Y) between the predetermined reference point and the front visual field center".

On the basis of this result, the correction-amount setting section 62 having a function of the position detecting section detects information concerning a relative positional relation, that is, information concerning the correction reference point CP' (X0+$\Delta$X, Y0+$\Delta$Y) for setting a position of the boundary portion between the front visual field region and the side visual field region or information concerning a position of the boundary portion between the front visual field region and the side visual field region based on the correction reference point CP', generates a positional relation detection signal concerning a detected result, and causes the memory 63, which is the storing section, to store the positional relation detection signal.

Thereafter, when the endoscope 2 is used, first, the correction-amount setting section 62 reads the information concerning the correction reference point CP' (X0+$\Delta$X, Y0+$\Delta$Y) for setting a position of the boundary portion between the front visual field region and the side visual field region or the information concerning a position of the boundary portion between the front visual field region and the side visual field region based on the correction reference point CP' stored in the memory 63, which is the storing section.

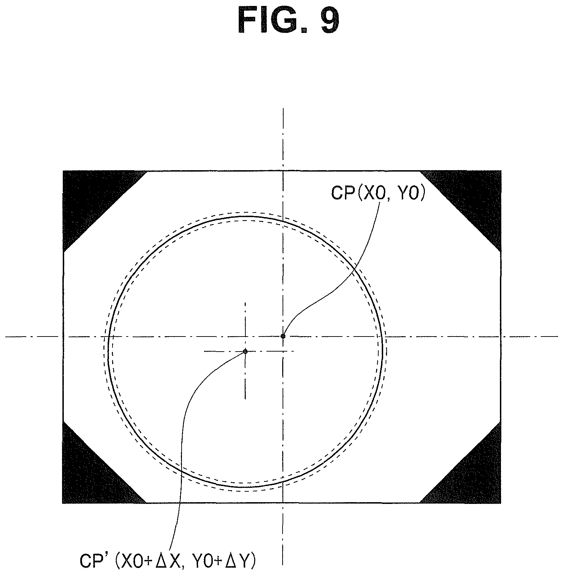

The image processing section 61 performs, on the basis of the correction reference point CP' (X0+$\Delta$X, Y0+$\Delta$Y) set by the correction-amount setting section 62, processing for, for example, moving a predetermined annular region, where image processing is performed, centering on the coordinate (X0, Y0) of the reference point CP of the image processing to an annular region centering on the coordinate (X0+$\Delta$X, Y0+$\Delta$Y) of CP', for example, as shown in Fig. 9.

Thereafter, while the endoscope 2 is used, as in the first embodiment, the image processing section 61 applies clarified image processing to the boundary portion (the boundary portion between the front visual field region and the side visual field region) on the basis of the information concerning the correction reference point CP' (X0+$\Delta$X, Y0+$\Delta$Y) set by the correction-amount setting section 62 and stored in the memory 63, which is the storing section, using the publicly-known method in Japanese Patent Application Laid-Open Publication No. 2013-066646 laid open by the applicant.

Further, the image processing section 61 performs predetermined image processing such as processing for extending respective parts of the first object-subject image and the second subject image and filling and reducing a frame-like edge formed in the boundary between the first subject image and the second subject image in the respective regions (the front visual field region and the side visual field region) on the basis of the correction reference point CP' (X0+$\Delta$X, Y0+$\Delta$Y) using, for example, the method in Japanese Patent Application Laid-Open Publication No. 2013-066646.

As explained above, with the endoscope system in the second embodiment, as in the first embodiment, it is possible to provide an endoscope system capable of independently and simultaneously observing a front visual field and a side visual field, the endoscope system being capable of precisely performing image processing even when axis deviation occurs between an optical axis of an objective optical system and a center axis of an image pickup device when an image pickup unit is assembled because of influence of machining accuracy of frames of the objective optical system and the image pickup device, irregularity during the assembly, or the like.

Further, in the second embodiment, it is possible to calculate the correction reference point CP' without setting position information of the boundary portion in advance during assembly. Therefore, compared with the first embodiment, it is unnecessary to set the position information during the assembly of the image pickup unit of the endoscope. It is possible to reduce assembly man-hour.

Note that, in the first embodiment, when the endoscope 2 is connected to the video processor 32 and displays an endoscopic image and the correction-amount setting section 62 once acquires the predetermined information (the information concerning "the shift amount ($\Delta$X, $\Delta$Y) between the predetermined reference point and the front visual field center") from the IC chip 64, the information is stored in, for example, the memory 63. Then, when it is determined that the same endoscope 2 is connected to the video processor 32 during use of the endoscope system 1 on a separate occasion with reference to peculiar information of the endoscopes stored in the IC chips 64 of the respective endoscopes 2, the correction-amount setting section 62 may set the correction reference point CP' (X0+$\Delta$X, Y0+$\Delta$Y) set before without reading the shift amount ($\Delta$X, $\Delta$Y) again.

Further, in the second embodiment, when the arithmetic unit 62a once calculates the shift amount ($\Delta$X, $\Delta$Y), similarly, the shift amount ($\Delta$X, $\Delta$Y) is stored in the memory 63 or the like. Then, when it is determined that the same endoscope 2 is connected to the video processor 32 during the use of the endoscope system 1 on a separate occasion, the correction-amount setting section 62 may set the correction reference point CP' (X0+$\Delta$X, Y0+$\Delta$Y) set before without reading the shift amount ($\Delta$X, $\Delta$Y) again.

The present invention is not limited to the embodiments explained above. Various changes, alternations, and the like are possible in a range in which the gist of the present invention is not changed.

For example, even if, for example, the information to the effect that image processing is performed on a predetermined annular region centering on the coordinate (X0, Y0) of the reference point CP of the image processing is not stored in the video processor 32, for example, the memory 63 in advance, it is also possible to detect the position of the boundary portion between the front visual field region and the side visual field region, designate a region having predetermined width as the image processing region directly from the boundary between the front visual field region and the side visual field region, and perform the predetermined image processing.

Further, in such an embodiment, information concerning a position of the image processing region calculated and designated from the boundary between the front visual field region and the side visual field region designated by the method explained above may be stored in, for example, the memory 63 by a method of, for example, converting the information into a coordinate.

By storing the information concerning the position of the image processing region in, for example, the memory 63, when it is determined that the same endoscope 2 is connected to the video processor 32 during the use of the endoscope system 1 on a separate occasion with reference to the peculiar information of the endoscopes generally stored in the IC chips 64 of the respective endoscopes 2, the information concerning the position of the image processing region set before may be set again.

As still another embodiment, in the first embodiment and the second embodiment, the position of the entire endoscopic image including the front visual field image and the side visual field image on which the predetermined image processing is performed may be further moved such that the center of the front visual field image approaches the coordinate (X0, Y0) of CP, which is the reference point of the image processing.

With the endoscope system of the present invention, it is possible to provide an endoscope system capable of independently and simultaneously observing a front visual field and a side visual field, the endoscope system being capable of precisely performing image processing even when axis deviation occurs between an optical axis of an objective optical system and a center axis of an image pickup device.

The present application is based upon and claims priority from Japanese Patent Application No. 2014-079766 filed in Japan on April 8, 2014, disclosed contents of which are incorporated in the specification and claims of the present application.

## Claims

1. An endoscope system comprising:

    an insertion section inserted into an inside of a subject;
    an image pickup section provided in the insertion section;
    a first optical system provided in the insertion section and configured to project a first subject image concerning a first region of the subject onto the image pickup section;
    a second optical system provided in the insertion section and configured to project a second subject image concerning a second region of the subject, at least a part of which is different from the first region, onto the image pickup section;
    an image generating section configured to generate an image based on the first subject image and the second subject image projected onto the image pickup section;
    a storing section configured to record information for setting a position of a boundary between the first subject image and the second subject image in the image; and
    an image processing section configured to perform predetermined image processing on the boundary between the first subject image and the second subject image based on the information for setting the position of the boundary.

2. The endoscope system according to claim 1, further comprising a position detecting section configured to detect a relative positional relation of a second position, which is a position where the first subject image is formed, with respect to a first position, which is a portion set in an image pickup region of the image pickup section, generate a positional relation detection signal concerning a detected result, and send the positional relation detection signal to the storing section, wherein
the image processing section determines, based on the positional relation detection signal, a range in which the image processing is performed on a region including a boundary portion between the first subject image and the second subject image, and performs the image processing.

3. The endoscope system according to claim 1, wherein
an image processing region subjected to image processing in the image processing section is set within an image pickup region, and
the image processing section moves the image processing region based on information concerning the position of the boundary between the first subject image and the second subject image in the image recorded in the storing section.

4. The endoscope system according to claim 3, wherein a frame-like designated range is set as the image processing region.

5. The endoscope system according to claim 3, wherein the image processing section further moves a position of an entire endoscopic image subjected to the image processing for moving the image processing region.

6. The endoscope system according to claim 1, wherein the image processing section applies image processing for reducing a frame-like edge formed in the boundary between the first subject image and the second subject image.

7. The endoscope system according to claim 6, wherein the image processing for reducing the edge by the image processing section is processing for extending each of parts of the first subject image and the second subject image and filling the edge.

8. The endoscope system according to claim 2, wherein the position detecting section reads numbers of pixels from a coordinate of the first position to the boundary between the first subject image and the second subject image in vertical and horizontal directions and detects the second position.

9. The endoscope system according to claim 8, wherein
when the numbers of pixels from the coordinate of the first position to the boundary between the first subject image and the second subject image in the horizontal direction are represented as Rr and R1 and the numbers of pixels from the coordinate of the first position to the boundary between the first subject image and the second subject image in the vertical direction are represented as Ru and Rd, and
when a shift amount in the horizontal direction between the coordinate of the first position and the second position is represented as ΔX and a shift amount in the vertical direction between the coordinate of the first position and the second position is represented as ΔY,
the position detecting section calculates the shift amounts ΔX and ΔY as follows:

$$\Delta X = |(Rr\text{-}Rl)/2|$$

$$\Delta Y = |(Ru\text{-}Rd)/2|$$

10. The endoscope system according to claim 1, wherein
the first subject image is a subject image in the first region including a front of the insertion section substantially parallel to a longitudinal direction of the insertion section,
the second subject image is a subject image in the second region including a side of the insertion section in a direction crossing the longitudinal direction of the insertion section,
the first optical system includes a front observation window for acquiring the subject image in the first region, and
the second optical system includes a side observation window for acquiring the subject image in the second region.

11. The endoscope system according to claim 1, wherein
the first optical system is disposed at a distal end portion in a longitudinal direction of the insertion section to be directed to a direction in which the insertion section is inserted,
the second optical system is disposed to surround a circumferential direction of the first optical system, and
the image pickup section is disposed to photoelectrically convert, on a same surface, the first subject image projected from the first optical system and the second subject image projected from the second optical system and is electrically connected to the image generating section.

**12.** The endoscope system according to claim 1, wherein the image generating section generates an image in which the first subject image has a substantially circular shape and the second subject image has an annular shape surrounding a circumference of the first subject image.

# FIG. 1

# FIG. 2

# FIG. 3

EP 3 103 378 A1

# FIG. 4

# FIG. 5

1

2

32

64 — IC CHIP

63 — MEMORY

34 — CCD

CORRECTION-AMOUNT SETTING SECTION — 62

60

IMAGE GENERATING SECTION

IMAGE PROCESSING SECTION — 61

OUTPUT

# FIG. 6

CP(X0, Y0)

C

ΔY

ΔX

CP'

# FIG. 7

OUTPUT

# FIG. 8

# FIG. 9

CP(X0, Y0)

CP' (X0+ΔX, Y0+ΔY)

# FIG. 10

EP 3 103 378 A1

# FIG. 11

101b 102c 102a 102A

101a 101A

## INTERNATIONAL SEARCH REPORT

| International application No. |
| --- |
| PCT/JP2015/060189 |

**A. CLASSIFICATION OF SUBJECT MATTER**
*A61B1/04(2006.01)i, A61B1/00(2006.01)i, G02B23/24(2006.01)n*

According to International Patent Classification (IPC) or to both national classification and IPC

**B. FIELDS SEARCHED**

Minimum documentation searched (classification system followed by classification symbols)
A61B1/00-1/32

Documentation searched other than minimum documentation to the extent that such documents are included in the fields searched
Jitsuyo Shinan Koho          1922-1996    Jitsuyo Shinan Toroku Koho    1996-2015
Kokai Jitsuyo Shinan Koho    1971-2015    Toroku Jitsuyo Shinan Koho    1994-2015

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | JP 2013-066646 A  (Olympus Medical Systems Corp.),<br>18 April 2013 (18.04.2013),<br>paragraphs [0029], [0034], [0037], [0042] to [0055]<br>& US 2014/0204187 A1     & WO 2013/047215 A1<br>& EP 2762059 A1 | 1,6,7,10-12<br>2-5,8,9 |
| A | JP 2013-066648 A  (Olympus Corp.),<br>18 April 2013 (18.04.2013),<br>entire text; all drawings<br>& US 2013/0076879 A1 | 1-12 |

| ☒ | Further documents are listed in the continuation of Box C. | ☐ | See patent family annex. |
| --- | --- | --- | --- |

\*   Special categories of cited documents:
"A"   document defining the general state of the art which is not considered   to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search<br>    12 June 2015 (12.06.15) | Date of mailing of the international search report<br>    23 June 2015 (23.06.15) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (July 2009)

**INTERNATIONAL SEARCH REPORT**

International application No.

PCT/JP2015/060189

C (Continuation). DOCUMENTS CONSIDERED TO BE RELEVANT

| Category* | Citation of document, with indication, where appropriate, of the relevant passages | Relevant to claim No. |
|---|---|---|
| A | WO 2011/055641 A1 (Olympus Medical Systems Corp.),<br>12 May 2011 (12.05.2011),<br>entire text; all drawings<br>& JP 4850315 B2      & US 2011/0282148 A1<br>& EP 2420178 A1 | 1-12 |

Form PCT/ISA/210 (continuation of second sheet) (July 2009)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5030675 B
- JP 4856286 B
- JP 4884567 B

- JP 2013066646 A
- JP 2014079766 A